# EUROPEAN PATENT SPECIFICATION

(11) **EP 3 220 969 B1**
(45) Date of publication and mention of the grant of the patent: **26.08.2020**
(21) Application number: 15832708.0
(22) Date of filing: 17.11.2015
(51) Int. Cl.: A61L 27/30, A61L 27/54

(54) **ANTIBACTERIAL OSSEOCONDUCTIVE THIN FILM FOR IMPLANT**
ANTIBAKTERIELLE OSSEOKONDUKTIVE DÜNNSCHICHT FÜR IMPLANTAT
FILM MINCE OSTÉOCONDUCTEUR ANTIBACTÉRIEN POUR IMPLANT

(30) Priority: 17.11.2014 TR 201413586
(43) Date of publication of application: 27.09.2017
(73) Proprietor: Dopa Ilac San. Tic. Ltd Sti., Istanbul (TR); Joanneum Research Forschungsgesellschaft mbH, 8010 Graz (AT)
(72) Inventor: INAL, Kadir, Istanbul (TR); LACKNER, Jürgen M., 8010 Graz (AT); WALDHAUSER, Wolfgang, 8010 Graz (AT); GÜMÜS, Serap, 41380 Izmit/Kocaeli (TR); POLAT, Seyda, 41380 Izmit/Kocaeli (TR); DALÇIK, Hakki, 41380 Izmit/Kocaeli (TR)
(74) Representative: Dilg, Haeusler, Schindelmann Patentanwaltsgesellschaft mbH
(86) International application number: PCT/TR2015/050182
(87) International publication number: WO 2016/080937

(56) References cited:
- US-A1- 2007 287 027
- US-A1- 2009 035 341

## Description

### Technical Field

This invention is pertinent to the optimization of the surface of the implant in contact with the bone, by coating a thin film strictly held on metal, ceramic or polymer base and functionally graded. Having a definite micro/nano topography, the film enhances the development of the bone on the implant (osteoconductive behavior) and in vivo adherence of the implant to the bone, and behaves as antibacterial by preventing in vivo bacterial colonization, formation of biofilm and inflammation.

### Infrastructure of the Invention

When an implant is placed in the bone, in terms of medicine, it is a necessity for the synthetic implant material to adhere to the bone fast and strictly. Acute inflammatory response and biologic response of the tissue of the bone under the local tissue trauma conditions, on a large scale, depend on the osseoconductivity of the material applied on the surface of the implant and the topography of the surface (described by the surface roughness). On optimum conditions, callus formed during acute inflammation phase is osseointegrated and forms a mineralized, natural bone-like structure. A sole very thin layer on the surface of the implant is mechanically weak. Despite this, bacterial infection in the section of the implant prevents the formation of the mineralized bone tissue. This situation not only gives serious ache and pain to the patients, but also increases the expenses of the therapy. In serious cases, removal of the prosthesis and repetition of the operation may be required. Statistical data shows that, in the USA, in the year of 2005, 8.4 % infection occurred in buttock implants and this figure will increase to 47.5 % with extrapolation in the year of 2013. Besides, because infection in the first operation cannot be described, success rate of the repetitive operations is also low (43 %). In consequence of bacterial infection, 5-11 % of the dental implants fail and their removals are required. For this reason, implants having antibacterial features become compulsory against multiple resistant bacteria in order to decrease the risk of nosocomial infection. In addition to this, obtaining fast and strict osseointegration with bioactive osseoconductive materials, as a result of quick recovery of the bone, it may reduce the expenses of the therapy on a large scale.

There are a large variety of biocompatible materials which are state-of-the-art products for the implants having constant, long term bone contact. Optimal bulk materials to be used between broken bones and damaged vertebral disks, or for load transmission from the bone to the synthetic articulations have elastic module (rigidity) similar to the natural tissue surrounding or taken substitute. This prevents the implant from loosening in long term applications. While Co-Cr alloys, stainless steels and ceramics (alumina, zirconia) fail in this matter, titanium and its alloys fulfil this requirements. Although rigidity of the biologic tissue is very low considering metals in renovation of vertebral disk, it is rather proximate to the polymers such as polyetheretherketone (PEEK). However, all these materials except titanium are bioinert and do not show any interactivity with the surrounding bone. After surgical intervention, required time period for the formation of strong bone-implant connection in these bioinert materials is very long according to for example, titanium implants and this cause the rehabilitation of the patient to delay. Hydroxyapatites, calcium phosphates, bioglasses, fluorides and fluorines, calcium nitrates, calcium carbonates, various absorbable polymers, silicon, magnesium, strontium, vanadium or lithium are bioactive materials enabling fast osseointegration. But all these bioactive based materials such as high bioactive based materials, absorbable polymer or bioceramics under mechanic loadings, fail as bulk implant materials either due to low rigidity, low toughness, or various medical reasons.

This kind of osseoconductive thin films applied on the surfaces of the bioinert bulk implant materials, fulfil both mechanical and medical requirements. Convenient surface modifications are possible with bioactive coating materials formed by the various physical and chemical methods such as plasma storage, plasma spray storage, and in vitro sedimentation. In the past, bioceramic coatings having high bioactivity, such as hydroxyapatites, calcium phosphates, bioglasses, etc., are studied on both scientifically and technologically. The biggest disadvantages of this kind of ceramic materials are their low toughness (low ductility). Local plastic deformation at the level of toughness and micron, prevents bone-implant connection materialized by delamination of the coating from the implant surface from getting lost. Generally, this problem becomes more important in the materials having low elastic module, such as polymers. Consequently, adherence problems intensively appear in ceramic coated polymers. Alternatively, pure bioactive materials and polymers or compounds are candidate materials for this type of surface modifications.

In addition to the strict (and fast) connection between bone and implant, inhibition of bacterial infection in the section of implant is an extremely important surgical necessity as well. Infection not only gives ache and pain to the patient, but also increases the expenses of the therapy. The greatest problem in nosocomial multi resistant bacteria, such as Staphylococcus aureus (MRSA) and epidermidis is pertinent to the formation of the biofilm that prevents the infiltration of the pharmaceutical antibiotic drugs. In such a case, because osseointegration is stopped by bacterial endotoxins, a quick surgical involvement becomes necessary in order to change the implant. Even in dental, bone trauma and articulation implants, this involvement does not lead to much negation, it carries risk of paralysis than can be formed in spinal and intervertebral disk implants.

With its topography (roughness, micro and nano texture), hydrophilicity, electric charge and chemical compound, the surface of the implant can be used in order to prevent the bacterial adherence and the biofilm formation. The surfaces which are rough and hydrophilic at the same time, are more vulnerable to the adherence and colonization. Even if modification of the chemical compound arouses more interest, only a section which is at the topmost and is 20 nm thick plays an active role in interactivity. Two approach have been approved: (1) porous (polymer) materials containing antibiotic medication, and (2) antibacterial (antimicrobial) metals. Advantage of the latter is its nondependence to the antibiotic resistance of the bacteria and films. Preferred materials are the metals having potential antibacterial (antimicrobial) characteristics and biocompatible (not damaging in the environment where it will be). This kind of metals are silver, zinc, niobium, tantalum, hafnium, zirconium, titanium, chrome, platinum, and gold. Despite this, these materials are only "potential antibacterial/antimicrobial", because they are not so reactive to be active antibacterial in elemental state. On the other hand, when ionized, metals have much stronger antibacterial/antimicrobial effect. Among these metals, silver in the state of ion or compound, is the most known and most commonly used antibacterial/antimicrobial metal. Although elemental silver has a sum of antibacterial/antimicrobial effect, generally in a lot of antibacterial/antimicrobial applications, due to specific interactivity (absorption) of silver with the cell/tissue, it displays very low reactivity: In ionic position, even in very low ppb concentrations, destroying cell walls of a lot of nosocomial bacteria (gram-positive and negative), microbes (germs, spores), fungi and viruses, silver displays a wide antibacterial/antimicrobial activity spectrum. In addition to this, in silver oxide form, it behaves as more antimicrobial according to the elemental silver.

US2009/035341 A discloses an antibacterial coating comprises a biocidal but non-cytotoxic layer as a further transport layer which is impregnated with an active agent. Preferably, the biocidal agent is an alloy of silver, zinc and copper.

US2007/287027 A discloses an antibacterial osseoconductive thin coating for a bone implant comprising a layer of antibacterial metal such as silver combined with a primary metal.The coating can comprise a first metal selected from cobalt-chrome, tantalum, titanium, platinum, zirconium, niobium, stainless steel, and alloys thereof.

### Explanatory Figures of the Invention

**Figure 1****:** Schematic view of the functionally graded coating
**Figure 2****:** Schematic view of the functionally graded layered coating

### Explanatory References of the Invention

**1.** A base having the surface roughness between the range of nanometer and micrometer
**2.** Pre-processed base surface enhancing the adherence of the film
**3.** Layer 1 displaying little osseoconductive behavior, having low bioresorption speeds and acceptable osseointegration
**4.** Layer 2 displaying high bioresorption and osseoconductivity, gradually alloyed with antibacterial/antimicrobial agent and able to be alloyed another agent in order to electrochemically control of the release speed of the osseoconductive and antibacterial/antimicrobial agents
**5.** Additionally, the surface roughness at the level of nanometer, controlled by the film growth conditions
**5.1.** Layer 3 comprising antibacterial/antimicrobial agent, and able to contain another agent controlling release speed electrochemically, and displaying high antibacterial/antimicrobial behavior

### Disclosure of the Invention

The invention is directed to an antibacterial osseoconductive thin film coating for an implant for use in medicine and veterinary surgery, the thin film coating having *in vivo* antibacterial and bone formation promoting characteristics and providing these characteristics with a functionally graded chemical composition, the thin film coating comprising at least one antibacterial agent and at least one agent promoting bioresorption and bone formation, the thin film coating being an alloyed thin film coating comprising minimum three different metals, wherein the at least one agent promoting bioresorption and bone formation is magnesium.

The invention is aimed to meet the continual need of the osseoconductive and antibacterial/antimicrobial materials able to adhere to any surface, having a controllable release speed and long duration, displaying longtime osteoblast adherence, and having no toxic effect in relevant practice. Having these characteristics and with a functionally graded compound, optimized surface topography (roughness), the thin films can be accumulated on a wide range of base material surfaces. Thereby, for implants this invention contains both state-of-art base materials optimized in terms of mechanical specifications (e.g. having a suitable elastic vitality with a feature able to replace the damaged tissue or to build a bridge on it), and the original surface coatings to be done onto these materials.

The purpose of this invention is to supply medical and surgical needs such as enabling antibacterial/antimicrobial ion concentration in lower values immediately after the implantation, in vivo quick release (burst release) of the antibacterial/antimicrobial agents and in following weeks, but more than required dose in order to prevent colonization in bacterial adherence and after. As a result, this agent is prevented from accumulating in definite sections of the body just as accumulation of silver in liver by consuming antibiotic agent source.

Alongside its antibacterial/antimicrobial behavior, the purpose of this invention is to have high osseoconductivity for the thin film to provide fast osseointegration during the first contact with the bone tissue.

Another purpose of the invention is to enable strict adherence of the implant surface which is medically necessary in long-term usage.

The purpose of the invention, high and fast adherence is controlled by the functionally graded chemical compound and bioresorption (bioactive) behavior of the film, and by the surface topography in the level of nanometer and micrometer.

In order to obtain all adherence and antibacterial/antimicrobial purposes and a high osseointegration, the thin film composition having high bioactivity, should provide both controlled release (bioresorption) of antibacterial/antimicrobial agent(s) and osseoconductive agent or agents in the process after implantation, and low release of these agents in next process.

Besides, the most important purpose of the invention is the provision of high adherence endurance onto base implant materials of the functionally graded film as well as among the layers of the multilayered structure and so prevention of the delamination. In addition to this, coating should have high cohesive endurance in order to prevent the thin film from fragmentizing in mechanical loads. This behavior becomes more important in case that base material is softer and more elastic.

Tested at 37°C in salty water, ion release speeds have been measured as 1x10⁻³ and 50 µg/mm²h and release speeds have been measured as 1x10-5 and 1x10-2 µg/mm²h. Besides, in thin film, antibacterial agent has a minimum concentration of 0.1 at-% and a maximum concentration of 95 at-%. The characteristic of the thin film is its being an antibacterial agent and at the same time, being alloyed with an additional element having a higher electrochemical standard electrode potential than the agent having bioresorption characteristic. The characteristic of the mentioned element is its minimum concentration of 0.1 at-% and its maximum concentration of 75 at-%. It develops implant osseointegration that promotes bioresorption and bone formation. The minimum concentration of the implant is 0.1 at-% and its maximum concentration is 99.6 at-%. The characteristic of the functionally graded chemical composition of the antibacterial osseoconductive thin film in the thickness between the range of 50 nm and 50 µm is its having a variable concentration gradient depending on the medical requirements.

In accordance with these purposes of the invention, present functionally graded multilayered coating and coating-base system should have following characteristics and structure:
- In order to enable the bone to adsorb mechanically, roughness of the base implant material (at the level of nanometer-micrometer), should be chosen by taking state-of-the art production (e.g. lustering, sandblasting) as a base.
- A convenient pre-processing duration should be chosen in order to provide the required adherence of the coating to the base surface. When thin film is accumulated with vacuum coating methods, surface cleaning improved with plasma or ion bundle, or activation process is applied (providing kinetic or ionic energy in the range of 5-5000 eV to vaporized particles such as inert and reactive metal or gas ions/atoms/molecules (e.g. gridded or ungridded source of ion and plasma, electrical glittering discharge, etc.)).
- For obtaining a functionally graded coating, displaying little osseoconductive behavior, having very low bioresorption speeds and an acceptable osseointegration, a soft metal such as titanium or zirconium is chosen because of their mechanical characteristicks and biocompatibleness for longterm adsorption of the bone after bioresorption of the accumulated layers on it (osteoblast adherence and callus formation).
- At least one metal is alloyed as graded in variable concentration onto the coating explained in previous item such as magnesium displaying high bioresorption and osteoconductivity, and promoting bone formation.
- Besides, a second element from antibacterial/antimicrobial group is alloyed as graded in variable concentration.
- In this coating, in order to electrochemically control the release speed of the osseoconductive and antibacterial/antimicrobial material, a third biocompatible element which is graded or in permanent concentration can be used.
- Surface roughness of the multi-layered, functionally graded film at the level of nanometer, is controlled by growth conditions.

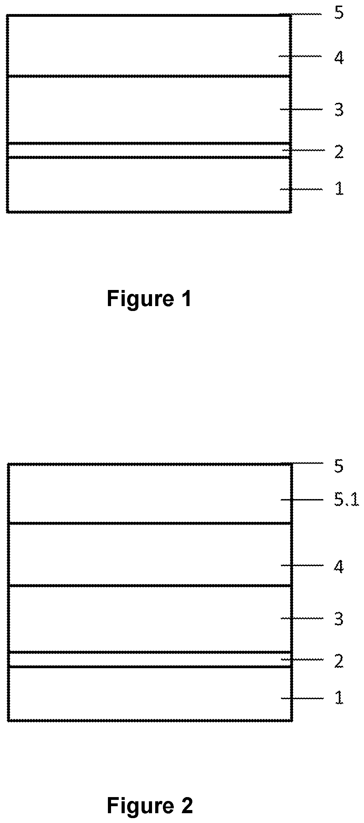

In this functionally graded coating, two different arrangements of the layers are given in Figure 1 and 2.

Functionally graded coating given in figure 1 comprises of a base having surface roughness in the range of nanometer and micrometer (1), preprocessed base surface enhancing adherence of the film (2), displaying little osseoconductive behavior, having low bioresorption speeds, and an acceptable osseointegration, the layer 1 (3), displaying high bioresorption and osteoconductivity, alloyed as functionally graded with antibacterial/antimicrobial agent, and able to be alloyed with another agent in order to electrochemically control the release speed of osseoconductive and antibacterial/antimicrobial agents, the layer 2 (4) and additionally, surface roughness in the range of nanometer and micrometer controlled by growth conditions (5).

Functionally graded coating given in figure 2 comprises of a base having surface roughness in the range of nanometer and micrometer (1), preprocessed base surface enhancing adherence of the film (2), displaying little osseoconductive behavior, having low bioresorption speeds, and an acceptable osseointegration, the layer 1 (3), displaying high bioresorption and osteoconductivity, alloyed as functionally graded with antibacterial/antimicrobial agent, and able to be alloyed with another agent in order to electrochemically control the release speed of osseoconductive and antibacterial/antimicrobial agents, the layer 2 (4), comprising an antibacterial/antimicrobial agent and able to contain another agent electrochemically controlling the release speed, displaying high antibacterial/antimicrobial behavior, the Layer 3 (5.1) and additionally, surface roughness in the range of nanometer and micrometer controlled by growth conditions (5).

After the implantation, osseoconductive and antibacterial/antimicrobial concentrations related to the defined thickness, provide fast release of the above stated antibacterial agent by the fast dissolution of the surface of the thin film in wet (isotonic) in vivo environment. Besides, it provides lower agent concentration in further process (over the antibacterial/antimicrobial action threshold) and with higher osseoconductive agent concentration, it allows fast bone formation, and high fastening strength between implant and bone. This situation, in the weeks following implant, assures the low release, thus preventing the adsorption of the migrating bacteria to the implant section from generally the other sections of the body.

Precise thickness of the chosen bioactive layer, functionally graded elemental compositions, and the thickness of the bottom layer displaying very low bioresorption, depends has not on the medical requirements. Any technique been described precisely for this functionally graded coating. Vacuum coating group contains physical vapor accumulation (physical vapor deposition, PVD) methods and chemical vapor accumulation (chemical vapor deposition, CVD) methods.

Disclosed process is suitable for the production of a great variety of appliances requiring controlled composition, however, it is more suitable for the screws in the contact with the bone, plates, implants such as nails for trauma surgery (fracture and bone alignment stabilization), tooth implants, vertebral disk prostheses, sensor and actuators placed in the bone, synthetic articulations, and synthetic organs. In addition to this, the invention is not restricted to only with these devices and it is able to be used in a large variety of other appliances in medical field as well. In general, this process is used in order to produce antibacterial/antimicrobial and osseoconductive products, or to develop the available products and raw materials.

Antibacterial osseoconductive thin film is able to be used in dental implants, vertebral disk implants, orthopedic implant, medical sensor, actuator implants and chip implants.

In another aspect, this invention is pertinent to the development of the antibacterial/antimicrobial and osseoconductive surfaces or other pieces of the medical devices used in human body, veterinary surgery and other practices.

## Claims

1. An antibacterial osseoconductive thin film coating for an implant for use in medicine and veterinary surgery,
the thin film coating having in vivo antibacterial and bone formation promoting characteristics and providing these characteristics with a functionally graded chemical composition,
the thin film coating comprising at least one antibacterial agent and at least one agent promoting bioresorption and bone formation,
the thin film coating being an alloyed thin film coating comprising minimum three different metals,
wherein the at least one agent promoting bioresorption and bone formation is magnesium.

2. The thin film coating according to claim 1, wherein the coating is functionally graded and comprises:
- a base (1) having surface roughness between the range of nanometer and micrometer,
- a pre-processed base surface (2) enhancing the adherence of the film,
- a layer 1 (3) displaying little osseoconductive behavior, having low bioresorption speeds and acceptable osseointegration,
- a layer 2 (4) displaying high bioresorption and osteoconductivity, alloyed with the antibacterial agent as functionally graded and optionally alloyed with another agent configured for electrochemically controlling the release speed of osseoconductive and antibacterial agents, and
- additionally the surface roughness (5) controlled by the film growth conditions at the level of nanometer,
or
- a base (1) having surface roughness between the range of nanometer and micrometer,
- a pre-processed base surface (2) enhancing the adherence of the film,
- a layer 1 (3) displaying little osseoconductive behavior, having low bioresorption speeds and acceptable osseointegration,
- a layer 2 (4) displaying high bioresorption and osteoconductivity, alloyed with the antibacterial agent as functionally graded and optionally alloyed with another agent configured for electrochemically controlling the release speed of osseoconductive and antibacterial agents,
- a layer 3 (5.1) comprising antibacterial agents and optionally another agent electrochemically controlling the release speed, displaying high antibacterial/antimicrobial behavior, and
- additionally the surface roughness (5) controlled by the film growth conditions at the level of nanometer.

3. The thin film coating according to claim 1 or 2, wherein ion release speeds of the at least one antibacterial agent tested in physiologic salty water in 37°C are 1 x10⁻³ and 50 µg/mm²h.

4. The thin film coating according to any one of claims 1 to 3, wherein the at least one antibacterial agent is selected from the group consisting of silver, zinc, niobium, tantalum, hafnium, zirconium, titanium, chrome, nickel, copper, platinum and gold.

5. The thin film coating according to any one of claims 1 to 4, wherein the at least one antibacterial agent has a minimum concentration of 0.1 at-% and a maximum concentration of 95 at-% in the thin film coating.

6. The thin film coating according to claim 1, wherein the thin film coating is alloyed with an antibacterial agent and at the same time, with another additional element having higher electrochemical standard electrode potential than the agent with bioresorption characteristic.

7. The thin film coating according to claim 6, wherein the element has a minimum concentration of 0.1 at-% and a maximum concentration of 75 at-%.

8. The thin film coating according to claim 6 or 7, wherein the element is selected from the group consisting of gold, platinum, palladium, copper and iron.

9. The thin film coating according to claim 1, wherein the at least one agent promoting bioresorption and bone formation enhances osseointegration and/or is osseoconductive.

10. The thin film coating according to any one of claims 1 to 9, wherein the at least one agent promoting bioresorption and bone formation has a minimum concentration of 0.1 at-% and a maximum concentration of 99.6 at-%.

11. The thin film coating according to claim 1, wherein the thin film coating provides a time controlled release of both the antibacterial agent and the agent promoting bioresorption and bone formation.

12. The thin film coating according to any one of claims 1 to 11, wherein the thin film coating has a total thickness within the range of 50 nm and 50 µm.

13. The thin film coating according to any one of claims 1, 6 or 12, wherein the thin film coating is deposited with any method among physical vapor, chemical vapor or spray accumulation onto
- surface of a pure, particle or fiber-reinforced polyetheretherketone (PEEK) polymer
- surface of a metal such as titanium and its alloys, steels, Co-Cr alloys, or magnesium
or
- surface of pure and zirconia, alumina, titanium oxides, silicon oxide, glass, a bioglass ceramic and glass.

14. The thin film coating according to any one of claims 1 to 13, wherein the thin film coating is suitable for dental implants, vertebral disk implants, orthopedic implants, medical sensor, actuator implants and chip implants.

## Patentansprüche

1. Eine antibakterielle osseokonduktive Dünnfilmbeschichtung für ein Implantat zur Verwendung in der Medizin und Veterinärchirurgie,
wobei die Dünnfilmbeschichtung *in vivo* antibakterielle und die Knochenbildung fördernde Eigenschaften hat und diese Eigenschaften mit einer funktionell abgestuften chemischen Zusammensetzung bereitstellt,
wobei die Dünnfilmbeschichtung mindestens ein antibakterielles Mittel und mindestens ein Mittel, das die Bioresorption und die Knochenbildung fördert, umfasst,
wobei die Dünnfilmbeschichtung eine legierte Dünnfilmbeschichtung ist, die mindestens drei verschiedene Metalle umfasst,
wobei das mindestens eine Mittel, das die Bioresorption und die Knochenbildung fördert, Magnesium ist.

2. Die Dünnfilmbeschichtung gemäß Anspruch 1, wobei die Beschichtung funktionell abgestuft ist und Folgendes umfasst:
- eine Basis (1) mit einer Oberflächenrauhheit zwischen dem Bereich von Nanometern und Mikrometern,
- eine vorverarbeitete Basisoberfläche (2), die die Haftung des Films verbessert,
- eine Schicht 1 (3), die ein geringes osseokonduktives Verhalten zeigt sowie eine niedrige Bioresorptionsgeschwindigkeit und eine akzeptable Osseointegration aufweist,
- eine Schicht 2 (4) mit einer hohen Bioresorption und Osteokonduktivität, die mit dem antibakteriellen Mittel funktionell abgestuft legiert ist und optional mit einem weiteren Mittel legiert ist, das konfiguriert ist, um die Freisetzungsgeschwindigkeit von osseokonduktiven und antibakteriellen Mitteln elektrochemisch zu steuern, und
- zusätzlich die Oberflächenrauheit (5), die durch die Filmwachstumsbedingungen auf das Niveau von Nanometern gesteuert wird,
oder
- eine Basis (1) mit einer Oberflächenrauhheit zwischen dem Bereich von Nanometern und Mikrometern,
- eine vorverarveitete Basisoberfläche (2), die die Haftung des Films verbessert,
- eine Schicht 1 (3), die ein geringes osseokonduktives Verhalten zeigt sowie eine niedrige Bioresorptionsgeschwindigkeit und eine akzeptable Osseointegration aufweist,
- eine Schicht 2 (4) mit einer hohen Bioresorption und Osteokonduktivität, die mit dem antibakteriellen Mittel funktionell abgestuft legiert ist und optional mit einem weiteren Mittel legiert ist, das konfiguriert ist, um die Freisetzungsgeschwindigkeit von osseokonduktiven und antibakteriellen Mitteln elektrochemisch zu steuern,
- eine Schicht 3 (5.1), die antibakterielle Mittel und optional ein weiteren Mittel umfasst, das die Freisetzungsgeschwindigkeit elektrochemisch steuert und ein hohes antibakterielles/antimikrobielles Verhalten zeigt, und
- zusätzlich die Oberflächenrauheit (5), die durch die Filmwachstumsbedingungen auf das Niveau von Nanometern gesteuert wird.

3. Die Dünnfilmbeschichtung gemäß Anspruch 1 oder 2, wobei die in physiologischem Salzwasser bei 37 °C getesteten Ionenfreisetzungsgeschwindigkeiten des mindestens einen antibakteriellen Mittels 1 x 10⁻³ und 50 µg/mm²h betragen.

4. Die Dünnfilmbeschichtung gemäß einem der Ansprüche 1 bis 3, wobei das mindestens eine antibakterielle Mittel aus der Gruppe, bestehend aus Silber, Zink, Niob, Tantal, Hafnium, Zirconium, Titan, Chrom, Nickel, Kupfer, Platin und Gold, ausgewählt ist.

5. Die Dünnfilmbeschichtung gemäß einem der Ansprüche 1 bis 4, wobei das mindestens eine antibakterielle Mittel eine Mindestkonzentration von 0.1 At.-% und eine maximale Konzentration von 95 At.-% in der Dünnfilmbeschichtung hat.

6. Die Dünnfilmbeschichtung gemäß Anspruch 1, wobei die Dünnfilmbeschichtung mit einem antibakteriellen Mittel und gleichzeitig mit einem weiteren zusätzlichen Element, das ein höheres elektrochemisches Standardelektrodenpotential hat als das Mittel mit der Bioresorptionseigenschaft, legiert ist.

7. Die Dünnfilmbeschichtung gemäß Anspruch 6, wobei das Element eine Mindestkonzentration von 0.1 At.-% und eine maximale Konzentration von 75 At.-% hat.

8. Die Dünnfilmbeschichtung gemäß Anspruch 6 oder 7, wobei das Element aus der Gruppe, bestehend aus Gold, Platin, Palladium, Kupfer und Eisen, ausgewählt ist.

9. Die Dünnfilmbeschichtung gemäß Anspruch 1, wobei das mindestens eine Mittel, das die Bioresorption und die Knochenbildung fördert, die Osseointegration verbessert und/oder osseokonduktiv ist.

10. Die Dünnfilmbeschichtung gemäß einem der Ansprüche 1 bis 9, wobei das mindestens eine Mittel, das die Bioresorption und die Knochenbildung fördert, eine Mindestkonzentration von 0.1 At.-% und eine maximale Konzentration von 99,6 At.-% hat.

11. Die Dünnfilmbeschichtung gemäß Anspruch 1, wobei die Dünnfilmbeschichtung eine zeitgesteuerte Freisetzung von sowohl dem antibakteriellen Mittel als auch von dem Mittel, das die Bioresorption und die Knochenbildung fördert, bereitstellt.

12. Die Dünnfilmbeschichtung gemäß einem der Ansprüche 1 bis 11, wobei die Dünnfilmbeschichtung eine Gesamtdicke im Bereich von 50 nm bis 50 µm hat.

13. Die Dünnfilmbeschichtung gemäß einem der Ansprüche 1, 6 oder 12, wobei die Dünnfilmbeschichtung mit einem Verfahren von physikalischem Dampf, chemischem Dampf oder Spray Akkumulierung abgeschieden wird auf
- eine Oberfläche von reinem, partikel- oder faserverstärktem Polyetheretherketon (PEEK) Polymer
- eine Oberfläche eines Metalls, wie zum Beispiel Titan und dessen Legierungen, Stahl, Co-Cr Legierungen oder Magnesium
oder
- eine Oberfläche von reinem Zirkonoxid, Aluminiumoxid, Titanoxiden, Siliciumoxid, Glas, einer Bioglaskeramik und Glas.

14. Die Dünnfilmbeschichtung gemäß einem der Ansprüche 1 der 13, wobei die Dünnfilmbeschichtung für Zahnimplantate, Bandscheibenimplantate, orthopädische Implantate, medizinische Sensoren, Aktuatorimplantate und Chipimplantate geeignet ist.

## Revendications

1. Un revêtement en film mince ostéoconducteur antibactérien pour un implant destiné à être utilisé en médecine et en chirurgie vétérinaire,
le revêtement en film mince ayant des caractéristiques antibactériennes et favorisant la formation osseuse in vivo et fournissant ces caractéristiques avec une composition chimique à gradation fonctionnelle,
le revêtement en film mince comprenant au moins un agent antibactérien et au moins un agent favorisant la biorésorption et la formation osseuse,
le revêtement en film mince étant un revêtement en film mince allié comprenant au minimum trois métaux différents,
ledit au moins un agent favorisant la biorésorption et la formation osseuse étant du magnésium.

2. Le revêtement en film mince selon la revendication 1, dans lequel le revêtement est à gradation fonctionnelle et comprend :
- une base (1) présentant une rugosité de surface comprise dans la gamme allant du nanomètre au micromètre,
- une surface de base prétraitée (2) améliorant l'adhérence du film,
- une couche 1 (3) présentant un faible comportement ostéoconducteur, présentant de faibles vitesses de biorésorption et une ostéointégration acceptable,
- une couche 2 (4) présentant une biorésorption et une ostéoconductivité élevées, alliée à l'agent antibactérien en tant que gradation fonctionnelle et éventuellement alliée à un autre agent configuré pour contrôler électrochimiquement la vitesse de libération d'agents ostéoconducteurs et antibactériens, et
- additionnellement, la rugosité de surface (5) contrôlée par les conditions de croissance du film à un niveau nanométrique,
ou
- une base (1) ayant une rugosité de surface située entre la gamme allant du nanomètre au micromètre,
- une surface de base prétraitée (2) améliorant l'adhérence du film,
- une couche 1 (3) présentant un faible comportement ostéoconducteur, présentant de faibles vitesses de biorésorption et une ostéointégration acceptable,
- une couche 2 (4) présentant une biorésorption et une ostéoconductivité élevées, alliée à l'agent antibactérien en tant que gradation fonctionnelle et éventuellement alliée à un autre agent configuré pour contrôler électrochimiquement la vitesse de libération d'agents ostéoconducteurs et antibactériens,
- une couche 3 (5.1) comprenant des agents antibactériens et éventuellement un autre agent contrôlant électrochimiquement la vitesse de libération, présentant un comportement antibactérien / antimicrobien élevé, et
- additionnellement, la rugosité de surface (5) contrôlée par les conditions de croissance du film à un niveau nanométrique.

3. Le revêtement en film mince selon la revendication 1 ou la revendication 2, dans lequel les vitesses de libération des ions dudit au moins un agent antibactérien testé dans de l'eau physiologiquement salée à 37°C sont de 1 x 10⁻³ et 50 µg / mm²h.

4. Le revêtement en film mince selon l'une quelconque des revendications 1 à 3, dans lequel ledit au moins un agent antibactérien est choisi dans le groupe constitué par l'argent, le zinc, le niobium, le tantale, le hafnium, le zirconium, le titane, le chrome, le nickel, le cuivre, le platine et l'or.

5. Le revêtement en film mince selon l'une quelconque des revendications 1 à 4, dans lequel ledit au moins un agent antibactérien a une concentration minimale de 0,1% at et une concentration maximale de 95% at dans le revêtement en film mince.

6. Le revêtement en film mince selon la revendication 1, dans lequel le revêtement en film mince est allié avec un agent antibactérien et, en même temps, avec un autre élément supplémentaire ayant un potentiel d'électrode standard électrochimique plus élevé que l'agent ayant une caractéristique de biorésorption.

7. Le revêtement en film mince selon la revendication 6, dans lequel l'élément a une concentration minimale de 0,1% at et une concentration maximale de 75% at.

8. Le revêtement en film mince selon la revendication 6 ou la revendication 7, dans lequel l'élément est choisi dans le groupe constitué par l'or, le platine, le palladium, le cuivre et le fer.

9. Le revêtement en film mince selon la revendication 1, dans lequel ledit au moins un agent favorisant la biorésorption et la formation osseuse améliore l'ostéointégration et / ou est ostéoconducteur.

10. Le revêtement en couche mince selon l'une quelconque des revendications 1 à 9, dans lequel ledit au moins un agent favorisant la biorésorption et la formation osseuse a une concentration minimale de 0,1% at et une concentration maximale de 99,6% at.

11. Le revêtement en film mince selon la revendication 1, dans lequel le revêtement en film mince fournit une libération contrôlée dans le temps à la fois de l'agent antibactérien et de l'agent favorisant la biorésorption et la formation osseuse.

12. Le revêtement en film mince selon l'une quelconque des revendications 1 à 11, dans lequel le revêtement en film mince a une épaisseur totale située dans la gamme allant de 50 nm et 50 µm.

13. Le revêtement en film mince selon l'une quelconque des revendications 1, 6 ou 12, dans lequel le revêtement en film mince est déposé par n'importe quel procédé parmi la vapeur physique, la vapeur chimique ou l'accumulation de pulvérisation sur
- la surface d'un polymère polyétheréthercététone (PEEK) pur, renforcé de particules ou de fibres,
- la surface d'un métal tel que le titane et ses alliages, les aciers, les alliages Co-Cr ou le magnésium,
ou
- la surface d'un verre pur et de zircone, d'alumine, d'oxydes de titane, d'oxyde de silicium, d'une céramique bioverre et d'un verre.

14. Le revêtement mince en film selon l'une quelconque des revendications 1 à 13, dans lequel le revêtement en film mince convient aux implants dentaires, aux implants de disque vertébral, aux implants orthopédiques, aux capteurs médicaux, aux implants actionneurs et aux implants à puce informatique.
